Europäisches Patentamt

European Patent Office

Office européen des brevets

(11) Publication number : **0 469 814 A1**

# (12) EUROPEAN PATENT APPLICATION

(21) Application number : **91306904.3**

(22) Date of filing : **29.07.91**

(51) Int. Cl.⁵ : **A61M 15/00**

(30) Priority : **31.07.90 GB 9016789**

(43) Date of publication of application :
**05.02.92 Bulletin 92/06**

(84) Designated Contracting States :
**AT BE CH DE DK ES FR GB GR IT LI LU NL SE**

(71) Applicant : **LILLY INDUSTRIES LIMITED**
**Erl Wood Manor**
**Windlesham Surrey GU20 6PH (GB)**

(72) Inventor : **Everett, Mervyn George**
**The Brae, Acrefield Road**
**Liverpool L25 9JN (GB)**

Inventor : **Murray, John Henry**
**20 Druids Park**
**Liverpool L18 3LJ (GB)**
Inventor : **Chatham, Sarah Marianna**
**72 Broomfield Avenue**
**Lightwater, Surrey (GB)**
Inventor : **Sandler, Susan Myra**
**Flat 6, 2 Langley Avenue**
**Surbiton, Surrey KT6 6QL (GB)**
Inventor : **Crowther, Terence Roger**
**'Bankdam', The Street, Eversley**
**Basingstoke, Hants (GB)**

(74) Representative : **Tapping, Kenneth George et al**
**Lilly Industries Limited Patent Department Erl**
**Wood Manor**
**Windlesham Surrey, GU20 6PH (GB)**

(54) **Medicament administering devices.**

(57)   A method of providing a unit dosage form of a finely divided solid medicament for inhalation by a patient is provided, which comprises engaging the base layer (40) of a container of the medicament to burst the lid layer (39) of the container (38) and discharge the medicament into a chamber for inhalation. The container may be in a blister pack of such containers, each blister containing up to 10mg of medicament. The container may be held in the first chamber (19) of a suitable device and a discharge element (32) may be used to burst the container and discharge its contents into a second chamber.

EP 0 469 814 A1

$\overline{FI_{\Xi}.1}$

This invention relates to a method and devices for use in administering medicaments to or by patients and in particular medicaments in finely divided solid form, and to blister packages for use therewith.

There is a need for a device for dispensing very small quantities of a medicament in powder form for inhalation by a patient requiring local or systemic drug treatment. Known devices used by asthma patients, for example, can be loaded with a blister package containing a day's supply of a drug in the form of single doses mixed with relatively large amounts of a carrier, such as lactose powder. A mechanism in the device pierces the walls of the blister and the patient then exerts suction to draw the powder, which falls into an air passage in the device, into his lungs. The carrier can act as an irritant to some patients when inhaled. Alternative devices use pressurised containers filled with a propellant. However, because of environmental considerations, such pressurised aerosol spray packs are losing favour, and may be banned in some countries.

The present invention avoids the disadvantages of the known methods of treating patients requiring systemic treatment by inhalation.

Accordingly, the first aspect of the invention comprises a method of providing a unit dosage form of a finely divided solid medicament for inhalation by a patient comprising engaging the base layer of a container of the medicament to burst the lid layer of the container and discharge the medicament into a chamber for inhalation. By "bursting" is meant the rupturing of the lid of a container by the application of suitable pressure to the base layer.

The container may be in a blister pack of such containers, which may for example be a strip of base and lid laminates with a series of blisters spaced there-along formed by curved areas of the base laminate. The blisters may be of regular or irregular shape and may, for example, be semi-spherical or semi-ovoid, with circular or non-circular cross section.

In general, each blister will contain up to 10mg of medicament, for example from about 100µg to 10mg. Typically the blisters will contain from about 500µg to about 2 mg of medicament.

The container may be held in the first chamber of a suitable device and a discharge element of the device used to burst the container and discharge its contents into a second chamber.

Thus according to a further aspect of the invention there is provided a device for use in the above method comprising a housing defining first and second zones, the second zone being a chamber, an aperture establishing communication between the zones, a discharge element operable in use to engage a base or blister layer of a blister container of medicament in the first zone and burst the lid layer of the container to discharge the medicament through the aperture into the second zone and an outlet from the second zone for passage of the discharged medicament to the patient by inhalation.

Preferably, the first zone is also a chamber, and the outlet from the second zone forms a mouthpiece for the patient to inhale the medicament. An air inlet may be provided in the second zone, preferably remote from the mouthpiece so that air is drawn past the aperture in use.

The aperture may be in a wall which engages the lid layer during bursting of the container.

The discharge element may have a flat surface for engaging the base layer.

The discharge element may have a curved surface for engaging the base layer.

The discharge element does not itself engage the lid layer of the blister.

There may be means operable to advance a strip of spaced blister containers to bring successive containers into position for bursting by the discharge element.

The advancing means may comprise a toothed wheel for engagement with spaced apertures in the strip.

There may be means operatively connecting the advancing means and the discharge element such that operation of the advancing means operates the discharge element.

The strip for use with the device may comprise a spiral roll, and the device may include a spiral wall for separating the adjacent coils of the strip roll.

The invention may be performed in serious ways and some specific embodiments with possible modifications will now be described by way of example with reference to the accompanying drawings, in which:

Fig. 1 is a section through a device for administering a medicament;
Fig. 2 is a perspective view of the device;
Fig. 3 is a plan view of part of a medicament blister strip;
Fig. 4 is a perspective view of another form of the device;
Fig. 5 is a vertical section through the device of Fig. 4 on an enlarged scale;
Fig. 6 is an exploded view of a strip housing;
Fig. 7 shows a blister strip ring;
Fig. 8 is a perspective view of a further form of the device;
Fig. 9 is a section on the line 9-9 of Fig 8;
Fig. 10 is a schematic diagram showing a part of the device of Fig. 8;
Figs. 11 and 12 illustrate bursting of a blister;and
Figs. 13 and 14 show forms of hammer head.

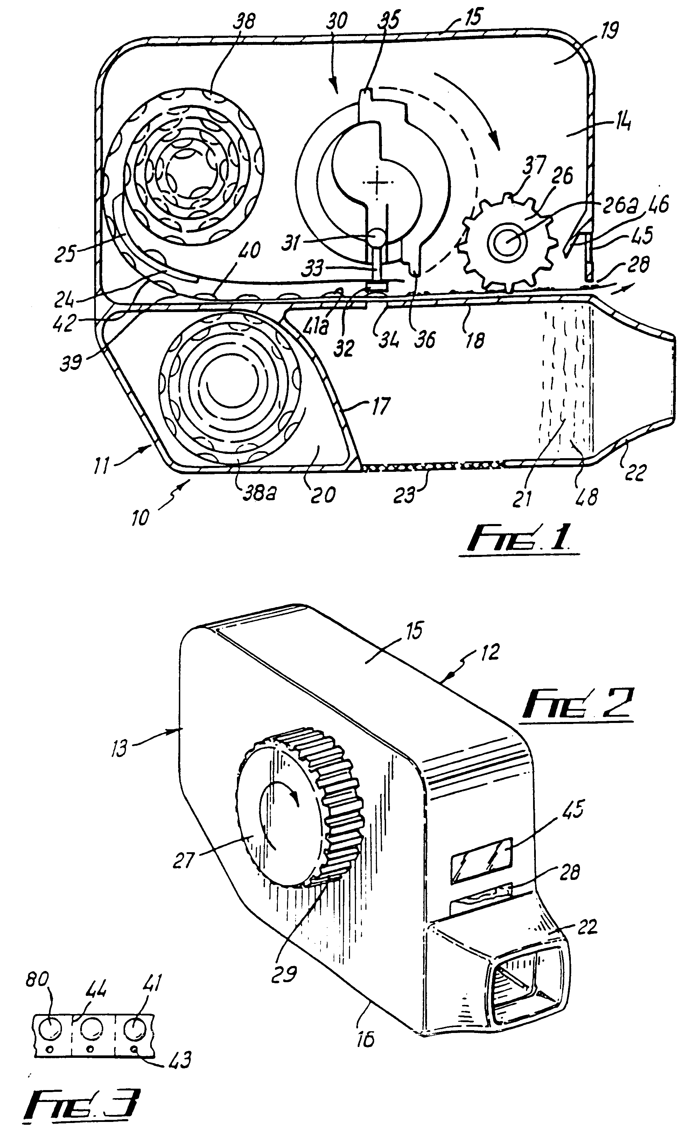

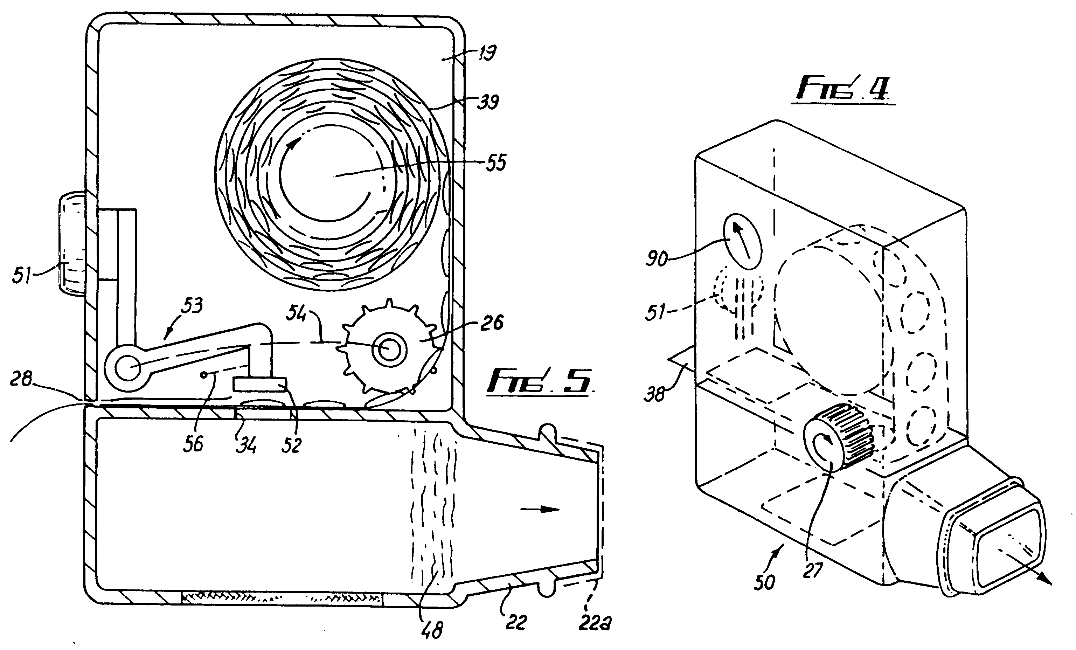

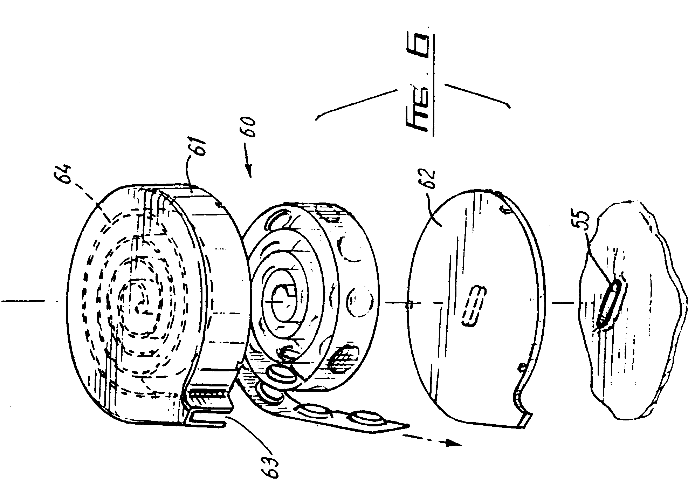

A device 10 (Figs. 1 to 3) for administering medicaments to patients comprises a plastics housing 11 formed from two parts 12, 13. The part 12 has base 14 and peripheral wall 15, while part 13 is in the form of a lid having a periphery 16 which is releasably engageable with the free periphery of the wall 15. The part 12 has internal walls 17, 18 providing compartments 19, 20, 21. The peripheral wall 15 is shaped to provide a mouthpiece 22 for insertion into a user's mouth and communicating with the compartment 21 which forms a medicament dispersion chamber. A filter or fine mesh screen 23 forms part of the wall 15 of the compartment 21 and admits air but keeps out unwanted solid matter such as dust.

In the compartment 19, a leaf spring 24 is secured at one end to a block 25 fixed to the base 14. A toothed wheel or sprocket 26 can rotate on an axle 26a secured to the base 14. The wheel 26 is adjacent an outlet slot 28 in the wall 15 adjacent the mouthpiece 22. An external rotatable operating knob 27 having a knurled periphery 29 is rotatably mounted in the lid 13 and has an operating stem extending into the compartment 19 and engageable with a cam device shown schematically at 30. A plunger or hammer 31 has a head 32 and a stem 33. The spring 24 engages the head 32 to bias the hammer 31 towards an aperture 34 in the wall 18 communicating with the compartment 21.

The cam device 30 includes projections 35, 36 which successively engage the teeth 37 of the wheel 26 on rotation of the knob 27. Rotation of the knob 27 through 180° causes projection 35 or 36 to rotate the wheel 26 through an angular distance of the spacing between two adjacent teeth, and to raise the hammer 31 against the action of the spring 24, and release the hammer for movement towards the aperture 34.

Mounted in the compartment 19 is a length 38 of material in the form of a strip wound in a spiral. The length 38 is in the form of a laminate of a generally flat strip 39 of a lid laminate on one side of which is a strip 40 of a base laminate secured to the base laminate and defining compartments or 'blisters' 41 with the base laminate. The form of construction of the base and lid laminates is known, the lid laminate including aluminium foil and the base laminate including a plastics layer, comprising, for example, PVC, polypropylene or similar plastics material or laminates. The lid layer may be scored to assist bursting. The compartments 41 contain the medicament which is to be administered. Each compartment 41 contains a measured amount of a powder for use in treating a respiratory condition.

The elongate length or blister strip 38 is placed with the free end portion 42 extending between the block 25 and the wall 15 and between the spring 24 and the wall 18. The strip 38 is supported on the wall 18, with the flat lid laminate 39 nearer the wall 18, and extends through the outlet slot 28 in the wall 15.

The strip 38 has apertures 43 for engagement with teeth 37. Thus as the wheel 26 is rotated one tooth, the strip 38 is moved forwards to move a blister 41 away from the hammer 31 and the next blister 41a into position under the hammer 31.

Thus in operation, a user places the mouthpiece 22 in his mouth, then rotates the knob 27 through a sufficient angle to activate the discharge element, in this case 180°, and inhales during rotation of the knob. Rotation of the knob 17 causes the hammer to move from an initial position under the action of the spring 24 to force the hammer head 32 into engagement with the blister 41 to burst the blister, forcing the powder through the aperture 34 into the compartment 21 and thus into the user's mouth. The hammer head 32 engages the curved surface 80 of the blister 41. On further rotation of the knob the hammer is then moved upwards against the action of the spring 24 to the initial position. The teeth 37 then move the strip 38 to bring the next blister 41a beneath the hammer head which acts as a discharge element. This procedure is repeated for each 180° rotation of the knob 27.

As shown in Fig. 11, the hammer head 32 initially engages the apex 81 of the symmetrical blister 01 and deform the blister causing the lid laminate 39 to burst in the region of the blister as shown dotted at 39a in Fig. 12. The medicament 82 is thus discharged in finely divided form from the blister. The head 32 does not directly engage the lid laminate. As shown the head 32 moves to a bursting position 32a (Fig. 11) spaced from the wall 18.

The hammer head 32 may initially engage the curved side of the blister 01, for example as shown in Fig 12, rather than the apex 81. The head 32 may for example be flat and circular (Fig. 11) or straight and flat (Figs. 12, 13) or may be curved (Fig. 10) so as to engage the surface 80 initially over an arcuate extent. Other forms are possible for the head 32.

The head 32 may be of a size fully to overlie the area of the blister 41 and of the aperture 34.

The compartment 21 may include means shown schematically at 48 for resisting or diminishing aggregation of the powder particles. Such means may comprise, for examle, baffles or venturis of other means for producing non-laminar air-flow.

The compartment 20 houses a fresh medicament strip 38a which can be placed in operating position in the compartment 19 when the strip 38 is used up.

The strip 38 may have lines of weakness provided by perforations 44 between the blisters 41 so that used portions can be torn off outside the housing 11.

An observation opening 45 is provided near the outlet 28 and an internal wall 46 provides a safety guard against interference with the wheel 26 from outside the housing 11.

Although it is preferred that the containers hold only the substantially pure medicament, the medicament may, where necessary, be mixed with suitable excipients such as a pharmaceutically acceptable carrier powder, preservatives, or other required additives.

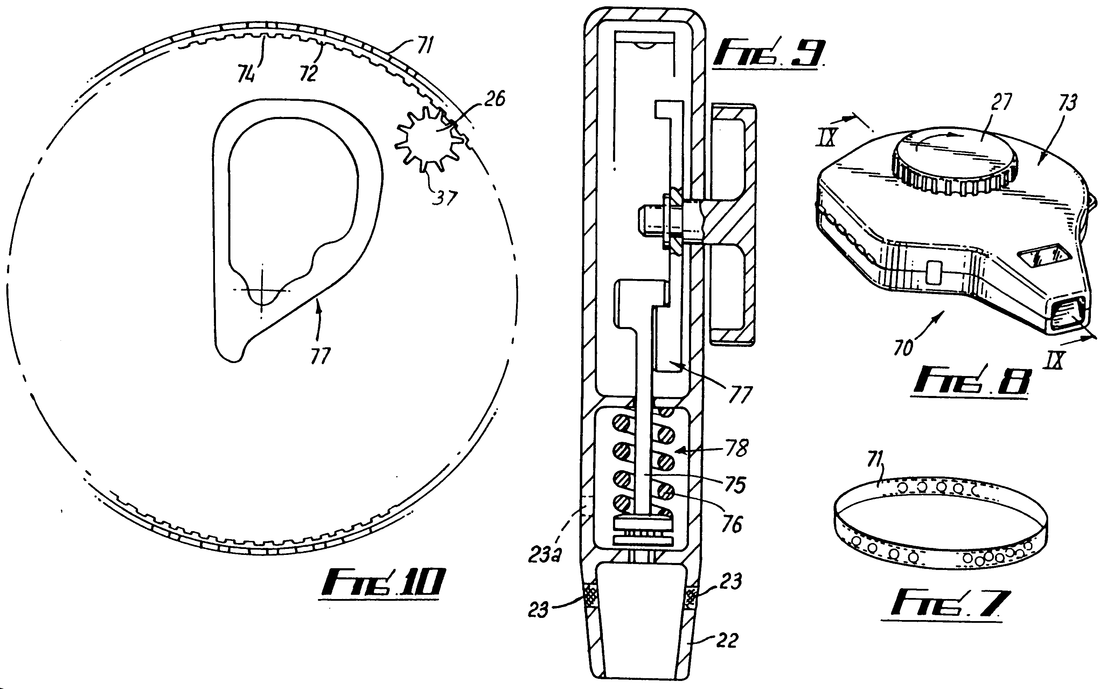

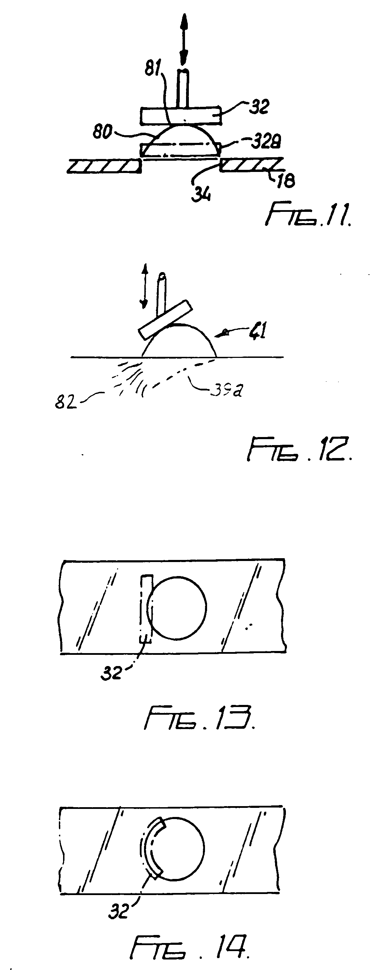

Figs. 4,5 show an alternative form of the device 50, wherein compartment 20 is omitted and the outlet slot 28 is directed away from the mouthpiece 22. In this case the knob 27 operates the wheel 26 directly.

An activation or discharge button 51 is connected to the discharge element in the form of the striker 52 by a linkage 53. Operation of the button 51 forces the striker 52 into engagement with the base laminate of the blister 41 to burst the blister lid laminate 39 as above and then raises the striker again, ready to burst the next blister when this has been fed forwards by the wheel 26.

In a modification, the wheel 26 is corrected by a linkage shown schematically at 54 to the striker 52 so that rotation of the knob 27 through an angular extent of one tooth raises the striker 52 against the action of a spring 56 so that subsequent operation of the button 51 releases the striker 52 which is urged by the spring 56 into engagement with the strip 38 to discharge the powder into the compartment 21.

A fresh strip 38 can be loaded in as required. If desired a stub block 55 extends from the base 14 and the strip 38 is supported on the block 55.

In a modification of the devices of Fig. 1 or Fig. 5 the strip 38 is housed in a cassette or container 60 having upper and lower parts 61, 62 defining an outlet 63. The part 61 preferably has a spiral internal wall 64 which separates adjacent coils of the strip spiral 38.

The compartment 19 has locating lugs for the cassette which can either be re-loaded with strip 38, or replaced, as required.

The compartment 19 in Fig. 1 or Fig. 5 could have an internal spiral wall 64 for receipt of the strip coil.

In any of the devices the housing 11 could include a removable wind-up spool on which the used strip 38 is wound during forward movement of the strip 38.

The device 70 of Figs. 7 to 10 uses a closed ring 71 of blister strip 38, mounted on a ring carrier 72 in a hollow housing 73. The ring 72 has grooves 74 engaged by teeth 37 on wheel 26 which is rotated by external knob 27. A hammer 75 is biased by a spring 76 and is raised by a cam mechanism 77 shown schematically and operatively linked to the knob 27. Rotation of the knob 27 through 360° causes the hammer to burst a blister and force the contents into the mouthpiece 22, raises the hammer against the action of the spring to ready the hammer for the next bursting, and moves the next blister into discharge position beneath the hammer.

Additional air openings 23a may communicate with the spring chamber 78.

In each case the mouthpiece 22 may have a removable and replaceable cover 22a (Fig. 5).

In some cases the 'mouthpiece' is adapted for insertion into anostril.

The device 50 (Fig. 4) includes a dose counter 90 which can be set to zero and indicates the number of doses which have been administered. The counter 90 is linked to the wheel 26 for movement therewith. A similar counter can be incorporated in the other embodiments.

The devices are light, portable, and may be sized for accommodation in a user's pocket.

In the above devices the medicament is discharged from the blister by bursting the lid layer and this provides a good discharge of the finely powdered medicament into the dispersion chamber 21 to form an aerosol for inhalation. The discharge does not depend on air flow produced by the user inhaling, the aerosol being readily and totally inhaled by only moderate suction exerted by the patient.

To ensure that the medicament is discharged at the time the patient is inhaling, a flap or flaps may be positioned over the air inlet, operatively connected to the discharge element release mechanism so that they open to allow inhalation by the patient through the device as the blister is burst and the aerosol formed.

Other forms of the invention will be readily apparent to those skilled in the art. Thus, for example, the discharge element may comprise a static fixed or rotatable element positioned above the aperture, forming a constricted path for the blister, and which engages the blister base. The blister is burst by the action of the patient in advancing the blister strip and bringing the next blister 41a into alignment with the aperture.

The device is useful in the treatment of patients who require local or systemic therapeutic or prophylactic treatment by inhalation of a solid medicament. By use of the device it is possible to provide accurate doses of pure medicament, avoiding the use of relatively large amounts of excipients which may irritate the respiratory tract. The resulting lower bulk made possible by avoiding excipients allows the compact device to carry a large number of doses. Thus at least 1 week's or more, preferably a month's supply may be stored within the device, i.e. from 100 to 400 or more containers and the need for frequent changes of blister pack is thus avoided.

Examples of medicaments which may be used with the device are bronchodilator compounds such as salbutamol, antiallergic compounds such as sodium cromoglycate and corticosteroids such as beclomethasone dipropionate, and medicaments for systemic use. The powder is very finely divided, substantially all less than 10 microns and a very high proportion less than 5 microns particle size. The powder does not fill the blister.

## Claims

1. A method of providing a unit dosage form of a finely divided solid medicament for inhalation by a patient comprising engaging the base layer of a container of the medicament to burst the lid layer of the container and discharge the medicament into a chamber for inhalation.

2. A method of claim 1 wherein a discharge element is brought into engagement with the base layer to burst the lid layer.

3. A method of claim 1 or claim 2 wherein the container is held in a first chamber and the medicament is discharged into a second chamber.

4. A method of any preceding claim wherein the container is part of an elongate strip comprising a plurality of such containers.

5. A method of any preceding claim wherein the medicament is substantially free from excipients.

6. A device for use in a method of claim 1 comprising a housing defining first and second zones, the second zone being a chamber, an aperture establishing communication between the zones, a discharge element operable in use to engage a base or blister layer of a blister container of medicament in the first zone and burst the lid layer of the container to discharge the medicament through the aperture into the second zone and an outler from the second zone for passage of the discharged medicament to the patient by inhalation.

7. A device of claim 6 wherein the outlet of the chamber is formed into a mouthpiece.

8. A device of claim 6 or claim 7 in which the first zone is a chamber.

9. A device of any one of claims 6 to 8 wherein there is an air inlet in the second zone.

10. A device of claim 9 wherein the air inlet is remote from the outlet such that, in use, air flows across the aperture.

11. A device of any one of claims 6 to 10 wherein the air outlet has a closure operably connected to the discharge element to open substantially simultaneously with the bursting of the container.

12. A device of any one of claims 6 to 11 wherein the aperture is in a wall which engages the lid layer during bursting of the container.

13. A device of any one of claims 6 to 12 wherein the discharge element has a flat surface for engaging the base layer.

14. A device of any one of claims 6 to 12 wherein the discharge element has a curved surface for engaging the base layer.

15. A device of any one of claims 6 to 14 also containing means operable to advance a strip of spaced containers to bring successive containers into position for bursting by the discharge element.

16. A device of claim 15 wherein the advancing means comprises a toothed wheel for engagement with spaced apertures in the strip.

17. A device of claim 15 or claim 16 wherein the advancing means and the discharge element are operatively connected such that operation of the advancing means operates the discharge element.

18. A device of any one of claims 6 to 17 having means to accept a cassette or cartridge containing a strip of containers.

19. A device of claim 18 which is capable of containing at least one weeks' supply of medicament containers.

20. A device of claim 19 which is capable of containing at least a month's supply of medicament containers.

21. A device of any one of claims 16 to 20 wherein the advancing means brings the discharge element into an operating position.

22. A device of any one of the claims 6 to 21 wherein registration means is provided, operatively connected to the advance means or discharge element to indicate the number of dosages used or remaining.

23. A blister pack for use in a device as claimed in any one of claims 6 to 22.

24. A blister pack of claim 23 in which a strip of containers in the form of blisters comprises a base layer having spaced bottom sections and a lid layer.

25. A blister pack of claim 24 wherein the lid layer is adapted to burst when pressure is applied to the base layer.

26. A blister pack of claim 25 wherein the blisters contain a finely divided solid medicament which does not completely fill the blister.

27. A blister pack of claim 26 wherein the blister also contains a gas or vapour inert to the medicament.

28. A blister pack of any one of claims 23 to 27 wherein the medicament is substantially free of solid excipients.

29. A blister pack of any one of claims 23 to 28 wherein the medicament particle size is less than about 10 microns.

30. A blister pack of any one of claims 23 to 29 wherein each blister contains from 100µg to 10mg of medicament.

31. A blister pack of claim 30 wherein each blister contains from 500µg to 2 mg of medicament.

32. A blister pack of any one of claims 23 to 31 comprising at least a weeks' supply of medicament.

33. A blister pack of claim 32 comprising at least one month's supply of medicament.

34. A blister pack of any one of claims 23 to 33 wherein the strip is provided with spaced apertures for use with advancing means.

35. A method of providing a unit dosage form according to claim 1 as herein described, with reference to any one of figures 1-14.

36. A device according to claims 6 as described herein, with reference to any one of figures 1-14.

37. A blister pack according to claim 23 as herein described, with reference to any one of figures 1-14.

_Fig. 1_

_Fig. 2_

_Fig. 3_

FIG. 4

FIG. 5.

EP 0 469 814 A1

FIG. 6

FIG. 9

FIG. 8

FIG. 10

FIG. 7

EP 0 469 814 A1

FIG. 11.

FIG. 12.

FIG. 13.

FIG. 14.

**European Patent Office**

# EUROPEAN SEARCH REPORT

Application Number

EP 91 30 6904

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | FR-A-2 516 387 (P.FABRE S.A.)<br><br>* page 6, line 4 - page 8, line 10; figure 3 * | 1-4,<br>6-10,12,<br>13-18,<br>21,<br>23-25,34 | A61M15/00 |
| Y | GB-A-2 169 265 (GLAXO GROUP LTD)<br><br>* page 1, line 39 - line 74 *<br>* page 1, line 112 - line 117 *<br>* page 2, line 49 - line 78 *<br>* page 2, line 126 - page 3, line 16 *<br>* figures 1,4 * | 1-4,<br>6-10,12,<br>13,15,<br>17,21 | |
| A | EP-A-0 129 985 (GLAXO GROUP LTD)<br><br>* the whole document * | 1-4,<br>6-10,12 | |
| P,X | WO-A-9 013 327 (RIKER LSBS INC)<br>* the whole document * | ALL | **TECHNICAL FIELDS SEARCHED (Int. Cl.5)**<br><br>A61M |
| Y | EP-A-0 315 951 (WARNER-LAMBERT CY)<br>* the whole document * | 23-25 | B65B<br>B65D |
| Y | FR-A-2 638 430 (J.CHOLLET)<br>* page 6, line 4 - line 10; figures 4,5 * | 14 | |
| Y | GB-A-2 108 390 (M.TAKAMORI)<br>* page 2, line 58 - line 66 * | 16,34 | |
| Y | EP-A-0 224 335 (M.COBB)<br>* page 3, line 27 - line 29; figure 2 * | 18 | |

The present search report has been drawn up for all claims

| Place of search | Date of completion of the search | Examiner |
|---|---|---|
| THE HAGUE | 31 OCTOBER 1991 | VEREECKE A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)